# EUROPEAN PATENT SPECIFICATION

(11) **EP 1 386 594 B1**
(45) Date of publication and mention of the grant of the patent: **12.09.2007**
(21) Application number: 03021906.7
(22) Date of filing: 17.01.1997
(51) Int. Cl.: A61F 13/02

(54) **Dressing mechanism**
Wundverband
Pansement

(30) Priority: 10.05.1996 US 17258 P
(43) Date of publication of application: 04.02.2004
(62) Divisional of application: 97904815.4
(73) Proprietor: Beaudry, Wallace J., Elkhart Lake, WI 53020 (US)
(72) Inventor: Beaudry, Wallace J., Elkhart Lake, WI 53020 (US)
(74) Representative: Sparing, Rolf Klaus

(56) References cited:
- FR-A- 928 389
- US-A- 3 085 024

## Description

The invention relates to a dressing mechanism in accordance to the preamble of claim 1.

FR 928,389 describes an dressing mechanism for the treatment of wounds, in particular to wounds which need to be contracted by an outer force, comprising a first and a second section each including an adhesive material and a third section coupled to the first section and the second section wherein the third section comprises an elastic material having three openings

US 5,116,675 describes an dressing mechanism for the treatment of wounds comprising a first section and a second section designed as adhesive strips, wherein a third section is coupled to the first section and the second section. The third section comprises an elastic material.

FR 1.203.376 describes an elastic dressing mechanism for the treatment of wounds, comprising a central bandage of rectangular shape which has adhesive stripes connected to ist border regions in order to fixate the bandage to the human skin.

US 3,085,024, which is considered as representing the closest prior art, describes a dressing mechanism for the treatment of wounds, comprising a bandage structure which is fastened by a first elastic material and a second elastic material each designed as a tape and each containing a plurality of perforations forming tear zones.

It is the object of the invention to provide a dressing mechanism, which permits a safe and persistent holding on an epidermal area.

The invention achieves this object for the dressing mechanism mentioned at the beginning by the characterizing features of claim 1.

With respect to the invention's applications as a dressing the invention may be generally described as comprising: a first section, a second section, and a third section. Of these three sections, the first section is coupled to the second section and the second section is coupled to the third section. The second section comprising an elastic material with the first section and the third section each having a first side; and a predetermined portion of the first side including an adhesive located thereon. Apart from the first and third section, the second section of the invention may also include a plurality of openings of a predetermined size and predetermined shape.

It should be noted that the predetermined shape or shapes of the openings may be spatially organized in a predetermined manner respective to each other. This is because in one embodiment of the present invention the second section is located between the first and third sections and is preferably composed of an elastic material. By,placing openings in the elastic material at predetermined locations the strength of the elastic material, when the elastic material is stretched, may be varied and the distribution of force across the elastic material may by varied. Also, the openings can be used to provide a visual reference to a user of the amount of stress being placed upon the second section and whether or not that section has been stretched sufficiently or been stretched too much since the shape of the openings will change in response to the degree to which the elastic material is stretched. Such a visual reference would be useful to medical personnel where, e.g., it is desirable for a predetermined amount of pressure to be applied to a wound.

Further, the second section includes a first margin and a second margin. The first section may be integral or coupled to the second section at the first margin; and the third section may be integral or coupled to the second section at the second margin.

Preferably, but not necessarily, the first section and the third section are laminated materials comprising a first layer, a second intermediate layer, and a third layer; with the third layer including the first side coated with adhesive and protected prior to use by a silicone release liner. The second section includes a first margin and a second margin. The first section includes a first channel located between the first layer and the third layer of the first section for receiving the first margin. The second section includes a second channel located between the first layer and the third layer of the second section for receiving the second margin. The second intermediate layer comprising an adhesive material. The first margin and the second margin of the second section respectively including at least one opening and the first margin engaging the second intermediate layer in the first channel and the adhesive material extending through the opening of the first margin; and the second margin engaging the second intermediate layer in the second channel and the adhesive material extending through the opening of the second margin.

The first and third layer of the first section and the first and third layer of the third section preferably being an inelastic material in some embodiments. The inelastic material may be of any suitable material such as a TYVEC brand type of material.

Alternatively, the dressing mechanism may be described as comprising: a first section, a second section, and a third section such that the first section is coupled to the second section and the second section is coupled to the third section. The first section and the third section comprising an elastic material and the first section and the third section each having a first side; and a predetermined portion of the first side including an adhesive located thereon.

Further the second section includes at least one opening of a predetermined size and the first section and the third section each include at least one opening comprising a predetermined shape. As previously noted the openings of predetermined shape are spatially organized in a predetermined manner respective to each other.

Also, the second section includes at least two margins and the first section and the third section each have a respective margin area. The first section margin is coupled to the second section at a first predetermined portion the margin of the second section. The third section margin being coupled to the second section at a second predetermined portion of the margin of the second section.

Preferably, the second section is a laminated material comprising at least a first layer, a second intermediate layer, and a third layer; the third layer including the first side. The first section and the third section including a first section margin and a third section margin. Both the first section margin and the third section margin being composed of an elastic material. The second section including at least one channel located between the first layer and the third layer of the second section at the second section margin for receiving the margins of the first and third sections. The second intermediate layer comprising an adhesive material. The first section margin and the third section margin respectively including at least one opening and the margins of the first and third sections engaging the second intermediate layer in the channel at the respective first predetermined margin area and second predetermined margin area so that the adhesive material extends through the openings formed in the material which makes up the first and third section margins. The first and third layer of the second section may, in this embodiment, comprises an inelastic material. The inelastic material may be a polyester.

Further, the second section includes at least one opening or at least one generally transparent section to either allow the wound or burn to be exposed to the air to be observed visually. Additionally, the second section could be modified to include a mechanism for irrigating the wound or burn under the bandage so that the wound or burn could be cleaned or treated without having to remove the dressing. Also, at least one side of the second section could be designed so that it is capable of isolating the wound in a clean environment by creating a solid antiseptic barrier around the wound through the use of a colloid type adhesive or be capable of contacting a wound or burn so that medicine could be applied to the wound or burn directly.
Figure 1 is a perspective view of a structure of the present invention. For the sake of simplicity the openings are not shown.
Figure 2 is a top plan view of the embodiment to the structure disclosed in Figure 1, now showing the openings.
Figure 3 is an illustration showing how an embodiment of the present invention may be used on an area of the human body that is subject to a high degree of movement.
Figure 4 is an illustration showing schematically how another alternative embodiment of the present invention may be used on an area of the human body that is subject to a high degree of movement.
Figure 5 illustrates how another alternative embodiment of the present invention may be used as a nasal dilator.
Figure 6 illustrates another method by which the alternative embodiment of the present invention shown in Figure 5 may be used as a nasal dilator.
Figure 7 illustrates how the embodiment shown in Figure 5 may be used to hold a flap of skin, in this case a human ear flap, in a predetermined position. This is useful where its is desired to have easy access to an area that might otherwise be blocked by a fold or flap of skin thus making work on that area difficult or cumbersome. For the sake of simplicity, the openings of the first and third section are not shown in figures 5 to 7.

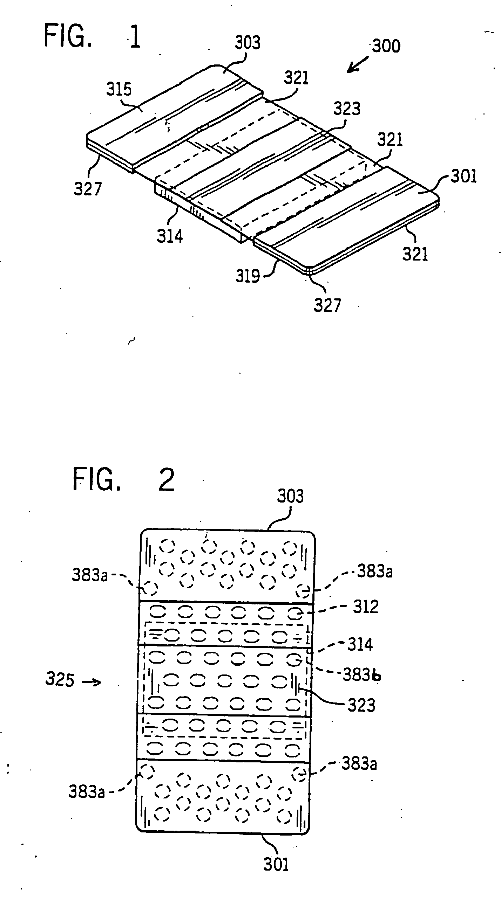

Referring now to Figures 1 and 2 an embodiment of the present invention may be observed. The dressing structure 300 is comprised of a multiple layer or laminated material at its anchor sections 301 and 303 and a latex rubber 321 at its second central section 325. The laminated material includes a top surface 315 made of TYVEC brand material and a bottom surface 319 also made of the same material but coated with a hypo-allergenic acrylic adhesive 327 and covered with a silicone release liner.

The center section 325 may be observed to include a TYVEC brand material stabilizing section 323 and a gauze pad 314. This creates a bandage or dressing structure which is suspended by the latex 321 between the anchoring sections 301 and 303. Further, the shape of the TYVEC top layer 323 need not be rectangular but can be of any design, e.g., round. When this embodiment is applied over a wound or other predetermined area of the epidermis the latex material 321 is stretched between the two anchoring sections 301 and 303 which causes the latex 321 to act much like a leaf spring and apply a positive pressure downward through the pad 314. Accordingly, the wound to which this device 300 is applied will have a positive pressure against it. It is well known in first aid that pressure applied to a wound will help reduce bleeding. The present invention thus provides an effective bandage which will also effectively limit bleeding from the wound. Further, the positive down pressure will effectively maintain contact of the pad 314 with the wound or other predetermined area despite movement of the surrounding epidermis.

The openings 312, shown in Figure 2, should also be noted. The openings 312 are located in a tension adjustment section of the latex 321. Depending upon the number of openings 312 or whether they are present at all the tension applied to the latex section 321. Further, as the tension adjustment section of the latex 321 is stretched to apply the dressing structure 300 the openings 312 will become distorted. The greater the stretching the greater the tension applied to the latex section 321. Consequently, a person applying the dressing structure disclosed herein may visually see the amount of tension applied to the latex section 321. This allows a person applying a dressing 300 or series of dressings 300 to apply the dressings 300 in a manner so that the pressure and exerted by the stretching of the latex 321 is kept relatively constant. Alternatively, it allows the user to apply dressings 300 which will apply a variety of pressures across the desired treatment area.

Referring again to Figure 1 a very simple version of the present invention is illustrated, when the openings are not shown. In this embodiment the dressing 300 is composed of a piece of latex 321. having two ends to which anchors 301 and 303 are respectively attached using an adhesive. The ends of the latex 321 are simply sandwiched between the layers 315 and 319. A piece of stiffening material 323 is glued across the mid-section of the latex 321 and pad 314 is glued to the underside of the latex 321 as illustrated. The bottom side of each respective anchor section 301 and 303 having an adhesive 327 applied thereto.

Figure 2 illustrates the embodiment of Figure 1 with the addition of a series of openings 383 being applied to the entire dressing 300. Depending upon the material through which the opening 383 is made the function of the opening will vary. Openings 312 in the latex 321 will act to vary the elasticity of the latex. Openings 383a will create stress points and help maintain the dressing 300 in a straight alignment between its anchors 301 and 303. Openings 383b will allow air access to the treatment area.

Additionally, it should be noted that a medicinal material could be applied to the elastic member over the portion of its surface which would be adjacent to the wound and thus aid in healing of the wound. Medicinal materials such as zinc chromate or calcium alginate or sodium alginate are possible such compounds.

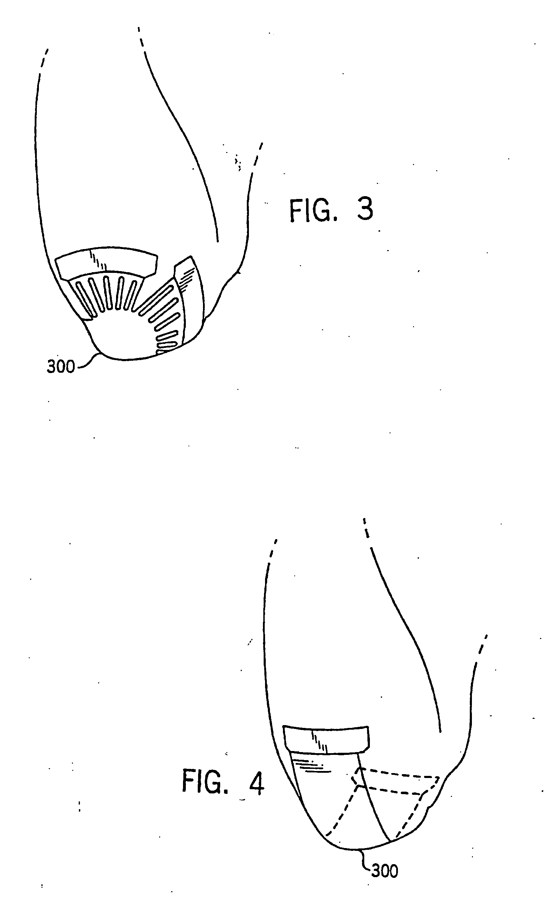

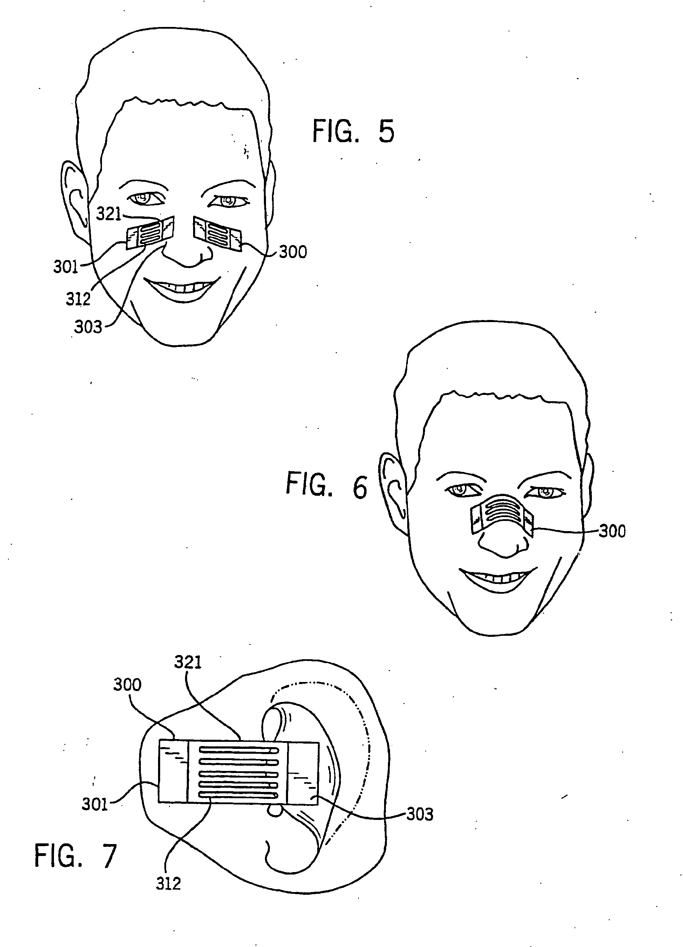

Referring now to Figures 3-7 various applications of the dressings 300 described herein may be seen to be illustrated in use on a human being.

The foregoing is considered as illustrative only of the principles of the invention. Furthermore, since numerous modifications and changes will readily occur to those skilled in the art, it is not desired to limit the invention to the exact construction and operation shown and described. While the preferred embodiment has been described, the details may be changed without departing from the invention, which is defined by the claims.

## Claims

1. A dressing mechanism, comprising
a first section (301), a second section (325) and a third section (303), the first section (301) being coupled to the second section (325) and the second section (325) being coupled to the third section (303);
wherein the first section (301) and the third section (303) each comprises an elastic material (321),
wherein the first section (301) and the third section (303) each have a first side including an adhesive (327) at least partially thereon, and
wherein the second section (325) includes at least a first margin and a second margin,
wherein the first section (301) and the third section (303) each include at least one opening (312, 383a),
**characterized in that** the first section (301) is coupled to the second section (325) in an integral manner, and
that the second section (325) is coupled to the third section (303) in an integral manner.

2. The dressing mechanism according to claim 1 wherein the second section (325) includes at least one further opening (312, 383b).

3. The dressing mechanism according to claim 1 or 2 wherein the second section (325) comprises laminated materials comprising at least a first layer (323) and a second layer (314) and wherein the first layer (323) including the first side, a portion of the first side having an adhesive coating.

4. The dressing mechanism according to claim 3, wherein at least a portion of said first section (301) is located between the first side of the first layer (323) and the second layer at the first margin, and at least a portion of said third section (302) is located between the first side of the first layer (323) and the second layer (314) at said second margin.

5. The dressing mechanism according to claim 4 wherein the first layer (323) of the second section (325) comprises an inelastic material.

6. The dressing mechanism according to claim 5 wherein the inelastic material is polyester.

7. The dressing mechanism according to one of claims 1 to 6 wherein the second section includes at least one generally transparent section.

8. The dressing mechanism according to one of claims 1 to 7 wherein the second section includes at least one wound irrigation mechanism.

9. The dressing mechanism according to one of claims 1 to 8 wherein the second layer of the second section (325) comprises a first side and a second side, and wherein the second side is capable of contacting a wound.

10. The dressing mechanism according to claim 9 wherein the second side comprises a medicinal material.

11. The dressing mechanism according to claim 10 wherein the medical material comprises zinc chromate.

12. The dressing mechanism according to claim 10 wherein the medical material comprises zinc chromate impregnated in a hydrocolloid material.

13. The dressing mechanism according to claim 10 wherein the medical material comprises an alginate.

14. The dressing mechanism according to claim 13 wherein the alginate comprises one of the group consisting of calcium alginate and sodium alginate.

## Patentansprüche

1. Verbandsmaterial, umfassend
einen ersten Abschnitt (301), einen zweiten Abschnitt (325) und einen dritten Abschnitt (303), wobei der erste Abschnitt (301) mit dem zweiten Abschnitt (325) verbunden ist und wobei der zweite Abschnitt (325) mit dem dritten Abschnit (303) verbunden ist,
wobei der erste Abschnitt (301) und der dritte Abschnitt (303) jeweils ein elastisches Material (321) umfassen,
wobei der erste Abschnitt (301) und der dritte Abschnitt (303) jeweils eine erste Seite aufweisen, welche wenigstens teilweise ein darauf befindliches Haftmittel (327) beinhaltet, und
wobei der zweite Abschnitt (325) wenigstens einen ersten Rand und einen zweiten Rand umfasst,
wobei der erste Abschnitt (301) und der dritte Abschnitt (303) jeweils wenigstens eine Öffnung (312, 383a) beinhalten,
**dadurch gekennzeichnet,**
**dass** der erste Abschnitt (301) mit dem zweiten Abschnitt (325) in integraler Art verbunden ist, und
**dass** der zweite Abschnitt (325) mit dem dritten Abschnitt (303) in integraler Art verbunden ist.

2. Verbandsmaterial nach Anspruch 1, wobei der zweite Abschnitt (325) wenigstens eine weitere Öffnung (312, 383b) beinhaltet.

3. Verbandsmaterial nach Anspruch 1 oder 2, wobei der zweite Abschnitt (325) ein geschichtetes Material umfasst, welches wenigstens eine erste Schicht (323) und eine zweite Schicht (314) umfasst, wobei die erste Schicht (323) die erste Seite beinhaltet, wobei ein Teil der ersten Seite eine haftende Beschichtung aufweist.

4. Verbandsmaterial nach Anspruch 3, wobei wenigstens ein Teil des ersten Abschnitts (301) an dem ersten Rand zwischen der ersten Seite der ersten Schicht (323) und der zweiten Schicht angeordnet ist, und
wobei wenigstens ein Teil des dritten Abschnitts (302) an dem zweiten Rand zwischen der ersten Seite der ersten Schicht (323) und der zweiten Schicht (314) angeordnet ist.

5. Verbandsmaterial nach Anspruch 4, wobei die erste Schicht (323) des zweiten Abschnitts (325) ein unelastisches Material umfasst.

6. Verbandsmaterial nach Anspruch 5, wobei das unelastische Material Polyester ist.

7. Verbandsmaterial nach einem der Ansprüche 1 bis 6, wobei der zweite Abschnitt wenigstens einen im Wesentlichen durchsichtigen Abschnitt umfasst.

8. Verbandsmaterial nach einem der Ansprüche 1 bis 7, wobei der zweite Abschnitt wenigstens einen Wundspülungsmechanismus umfasst.

9. Verbandsmaterial nach einem der Ansprüche 1 bis 8, wobei die zweite Schicht des zweiten Abschnitts (325) eine erste Seite und eine zweite Seite umfasst, und wobei die zweite Seite für das Berühren einer Wunde ausgebildet ist.

10. Verbandsmaterial nach Anspruch 9, wobei die zweite Seite ein medizinisches Material umfasst.

11. Verbandsmaterial nach Anspruch 10, wobei das medizinische Material Zink-Chromat umfasst.

12. Verbandsmaterial nach Anspruch 10, wobei das medizinische Material Zink-Chromat umfasst, das in einem hydrokolloiden Material imprägniert ist.

13. Verbandsmaterial nach Anspruch 10, wobei das medizinische Material ein Alginat umfasst.

14. Verbandsmaterial nach Anspruch 13, wobei das Alginat eines umfasst aus der Gruppe bestehend aus Calcium-Alginat und Natrium-Alginat.

## Revendications

1. Pansement, comprenant
une première section (301), une deuxième section (325) et une troisième section (303), la première section (301) étant couplée à la deuxième section (325) et la deuxième section (325) étant couplée à la troisième section (303),
la première section (301) et la troisième section comprenant respectivement un matériau élastique (321),
la première section (301) et la troisième section (303) ayant respectivement un premier côté sur lequel est placé au moins en partie un adhésif (327), et
la deuxième section (325) incluant une première marge et une deuxième marge,
la première section (301) et la troisième section (303) incluant respectivement au moins une ouverture (312, 383a),
**caractérisé en ce**
**que** la première section (301) est couplée à la deuxième section (325) de manière intégrale, et
**que** la deuxième section (325) est couplée à la troisième section (303) de manière intégrale.

2. Pansement selon la revendication 1, la deuxième section (325) incluant au moins une ouverture (312, 383b) supplémentaire.

3. Pansement selon la revendication 1 ou 2, la deuxième section (325) comprenant un matériau laminé comprenant au moins une première couche (323) et une deuxième couche (314), ladite première couche comprenant le premier côté, une portion du premier côté comprenant un revêtement adhésif.

4. Pansement selon la revendication 3, au moins une portion de la première section (301) étant située entre le premier côté de la première couche (323) et la deuxième couche au niveau de la première marge, et au moins une portion de la troisième section (302) étant située entre le premier côté de la première couche (323) et la deuxième couche (314) section au niveau de la deuxième marge.

5. Pansement selon la revendication 4, la première couche (323) et la deuxième couche (325) comprennent un matériau non-élastique.

6. Pansement selon la revendication 5, le matériau non-élastique étant un polyester.

7. Pansement selon l'une des revendications 1 à 6, la deuxième section incluant au moins une section généralement transparente.

8. Pansement selon l'une des revendications 1 à 7, la deuxième section incluant au moins un mécanisme pour irriguer la blessure.

9. Pansement selon l'une des revendications 1 à 8, la deuxième couche de la deuxième section (325) comprenant un premier côté et un deuxième côté, le deuxième côté étant capable de contacter une blessure.

10. Pansement selon la revendication 9, le deuxième côté comprenant un matériau médicinal.

11. Pansement selon la revendication 10, le matériau médicinal comprenant du chromate de zinc.

12. Pansement selon la revendication 10, le matériau médicinal comprenant du chromate de zinc imprégné dans un matériau hydrocolloïde.

13. Pansement selon la revendication 10, le matériau médicinal comprenant un alginate.

14. Pansement selon la revendication 13, l'alginate comprenant un produit dans le groupe consistant en l'alginate de calcium et l'alginate de sodium.
